(19) Europäisches Patentamt
European Patent Office
Office européen des brevets

(11) EP 1 218 889 B1

(12) # EUROPEAN PATENT SPECIFICATION

(45) Date of publication and mention of the grant of the patent:
**02.12.2009 Bulletin 2009/49**

(21) Application number: **00992052.1**

(22) Date of filing: **27.09.2000**

(51) Int Cl.:
***G12B 1/00*** *(2006.01)*

(86) International application number:
**PCT/IN2000/000094**

(87) International publication number:
**WO 2001/022791 (05.04.2001 Gazette 2001/14)**

(54) **CONTROLLED RELEASE COMPOSITIONS COMPRISING NIMESULIDE**

ZUSAMMENSETZUNGEN MIT KONTROLLIERTER FREIGABE ENTHALTEND NIMESULID

COMPOSITIONS A LIBERATION CONTROLEE CONTENANT DE LA NIMESULIDE

(84) Designated Contracting States:
**AT BE CH CY DE DK ES FI FR GB GR IE IT LI LU MC NL PT SE**

(30) Priority: **28.09.1999 IN DE129799**

(43) Date of publication of application:
**03.07.2002 Bulletin 2002/27**

(73) Proprietor: **Panacea Biotec Limited**
**110 044 New Dehli (IN)**

(72) Inventors:
- **SINGH, Amarjit**
**Panacea Biotec Limited**
**New Delhi 110 044 (IN)**
- **JAIN, Rajesh**
**Panacea Biotec Limited**
**New Delhi 110 044 (IN)**

(74) Representative: **Matthews, Derek Peter et al**
**Frank B. Dehn & Co.**
**St Bride's House**
**10 Salisbury Square**
**London**
**EC4Y 8JD (GB)**

(56) References cited:
**EP-A- 1 005 865 EP-A- 1 005 865**
**ES-A- 2 129 010 ES-A- 2 129 010**
**US-A- 4 666 928**

- **MANNA ET AL: "Design and evaluation of an oral controlled release microparticulate drug delivery system of nimesulide by ionotropic gelation technique and statistical optimization by factorial analysis" JOURNAL OF SCIENTIFIC AND INDUSTRIAL RESEARCH, XP001064400**
- **NAGOJI K.E.V. ET AL: "Release studies of nimesulide from ethyl cellulose and ethyl cellulose and hydroxy propyl methyl cellulose matrices." INDIAN JOURNAL OF PHARMACEUTICAL SCIENCES, (2000) 62/6 (482-484). , XP001063992**
- **GUO ET AL: "Preparation and in vitro dissolution of nimesulide sustained - release tablets" CHEMABS, XP002148832**
- **A. MANNA, I GHOSH, N GOSWANNI, L K GHOSH, B K GUPTA: "Design and evaluation of an oral controlled release microparticulate drug delivery system of Nimesulide by ionotropic gelation technique and statistical optimization by factorial analysis" JOURNAL OF SCIENTIFIC AND INDUSTRIAL RESEARCH, vol. 58, September 1999 (1999-09), pages 717-722, XP001064400**
- **DATABASE CAPLUS CHEMICAL ABSTRACTS SERVICE, COLUMBUS, OHIO, US; 2000, G. SHUGANG, C. BANGYING, Z. HANPING: "Preparation and in vitro dissolution of Nimesulide sustained release talets" XP002148832**
- **K. NAGOJI, S. SRINIVASA, M. BHANOJI RAO: "Release studies of Nimesulide from ethylcellulose and ethyl cellulose and hydroxypropyl methyl cellulose matrices" INDIAN JOURNAL OF PHARMACEUTICAL SCIENCES, 2000, pages 482-484,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 218 889 B1

## Description

The present invention relates to a controlled release composition of Nimesulide. The composition is related to a once-a-day dosage forms which are very useful in treatment of chronic diseases such as arthritis.

## TECHNICAL BACKGROUND OF THE INVENTION

Nimesulide is a nonsteroidal anti-inflammatory drug (NSAID) that also has antipyretic and analgesic properties. The compound is weakly acidic (pKa = 6.5) and differs from other NSAIDs in that its chemical structure contains a sulfonanilide moeity as the acidic group. (fig. 1) (Magni E, Nimesulide an overview, Drug Invest 1991; 3 Suppl. 2: 1-3).

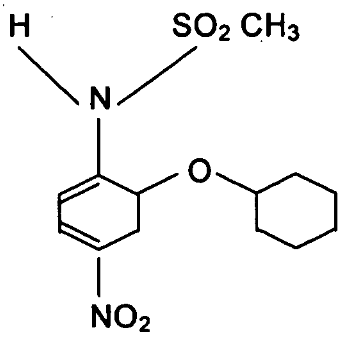

Fig. 1

The therapeutic effects of NSAIDs are largely the result of their ability to inhibit prostaglandin synthesis via inhibition of cyclooxygenase. Unfortunately, this effect is also responsible for the inhibition of gastroprotective prostaglandins, which leads to gastrointestinal intolerance.

In vitro, Nimesulide is a relatively weak inhibitor of prostaglandin synthesis and appears to exert its effects through a variety of mechanisms. (Magni E. The effect of nimesulide on prostanoid formation. Drugs 1993; 46 Suppl. 1: 10-4.) Indeed, the mechanism of action of this drug is more complex than previously thought and may involve interference with the production/action of mediators other than prostaglandins such as enzymes, toxic oxygen derivatives, cytokines, platelet-activating factor (PAF) and histamine.

The anti-inflammatory, analgesic and antipyretic activities of Nimesulide, a non-steroidal anti-inflammatory drug (NSAID) of the sulfonanilide class, have been demonstrated in a number of experimental models and in numerous clinical trials. Nimesulide has exhibited potency similar to or greater than that of indomethacin, diclofenac, piroxicam and ibuprofen in standard animal models of inflammation such as carrageenan-induced rat paw oedema and inflammation, ultraviolet light-induced erythema in guinea-pigs and adjuvant arthritis in rats. The analgesic potency in nimesulide was similar to that of ibuprofen and less than that of indomethacin in an acetic acid writhing test in rats, and acetic acid and acetycholine writhing tests in mice. Nimesulide has shown superior antipyretic potency to indomethacin, ibuprofen, aspirin and paracetamol (acetaminophen) in rats with yeast-induced fever.

Nimesulide is a relatively weak inhibitor of prostaglandin synthesis in vitro and appears to exert its effects through a variety of mechanisms including free-radical scavenging, effects on histamine release, the neutrophil myeloperoxidase pathway, bradykinin activity, tumour necrosis factor-$\alpha$ release, cartilage degradation, metalloprotease synthesis, phosphodiesterase type IV inhibition, platelet aggregation and synthesis of platelet activating factor. Animal studies have suggested that Nimesulide is less ulcerogenic than aspirin, indomethacin, naproxen, piroxicam and ibuprofen. Nimesulide appears to have little effect on renal prostaglandin synthesis in rats.

After oral administration of nimesulide 50 to 200 mg to healthy adult volunteers, peak serum concentrations of 1.98 to 9.85 mg/L are achieved within 1.22 to 3.17 hours. Compared with values obtained with oral drug administration, peak serum concentrations are slightly lower (2.14 to 2.32 mg/L) and are achieved more slowly (3 to 4.58 h) after rectal administration of nimesulide 100 and 200 mg. Oral drug absorption is nearly complete and concomitant administration of food may decrease the rate, but not the extent of absorption of nimesulide. The drug is extensively bound (99%) to plasma proteins and has an estimated apparent volume of distribution of 0.19 to 0.35 Ukg following oral administration.

In children, nimesulide suspension, granules and suppositories are more effective than placebo and are at least as effective as paracetamol, diclofenac, naproxen, lysine acetylsalicylate, mefenamic acid, ketoprofen and dipyrone in reducing the pain, inflammation and fever associated with respiratory tract infection, postoperative pain and musculoskeletal injury. Nimesulide has been well tolerated by both young and elderly adults and children in 2 large postmarketing surveillance surveys. As with other NSAIDs, the most common adverse effects are gastrointestinal disturbances (epigastralgia, heartburn, nausea, diarrhoea and vomitings; 5.1 to 8.5% of patients), dermatological reactions (rash, pruritus; 0.2 to 0.6%) and central nervous system effects (dizziness, somnolence, headache; 0.3 to 0.4%). Withdrawal rates associated with short term (up to 30 days) nimesulide treatment range from 1.1 to 2.2% in adult, elderly and paediatric patients.

Available data indicate that the gastrointestinal tolerability of nimesulide in adults and children is similar to that of other NSAIDs. The rate of endoscopically verified gastroduodenal irritation with nimesulide appears to be similar to that with placebo and diclofenac and less than that with indomethacin. The drug is well tolerated by most patients intolerant of aspirin and/or other NSAIDs and by patients with asthma.

The literature surveys shows that different dosage forms reported for nimesulide are tablets, granules, suppositories and suspension (Drugs 48 (3): 431-454, 1994) and lately our group has patented transdermal (US Pat. No. 5688829) and intramuscular injection (US Pat. No. 5716609) formulations and a nimesulide/cetirizine formulation including bilayer tablets thereof (Canadian Pat. Appln. No. 2253061). The reported dosage forms have to be administered twice-a-day based on biological half life of nimesulide. The usual oral/rectal dosage of nimesulide in adults is 100 to 200 mg twice daily, orally. For treatment of chronic diseases like arthritis the twice daily dosing regimen is difficult to comply with.

One approach to improve the possible non-compliance with the regimen is to develop controlled release dosage form for nimesulide The once-a-day dosage form is expected to significantly increase the dosing convenience and patient compliance. However, controlled release once-a-day dosage form of nimesulide have not been reported so far.

Controlled release compositions for oral use in the form of matrix type monolithic tablets, beads, capsules and coated tablets are known. However poorly soluble drugs like nimesuitde are known to give erratic and variable release under in-vivo conditions from such dosage forms.

One approach to formulate modified release dosage forms of NSAIDs is described in PCT Pub. No. WO9912524, wherein a unit dosage form comprising two fractions (i) a first quick release fraction, and (ii) a second fraction containing coated delayed release multiple units is described. However, such dosage forms having different fractions and coated multiple units are difficult to prepare and very cost intensive Moreover compression of such coated multiple units into tablets cause fracturing of the coat layer, thereby causing loss of reproducibility.

U.S. Pat. No. 5788987 Busetti et al. describes a time-specific controlled release dosage form. Such dosage forms are designed to provide delayed release of the active ingredient rather than extended release. Such formulations are not suitable for day long management of the disease.

Spanish Patent No. ES2129010 Oscar Gold describes the method of manufacturing of granules having prolonged action. The claimed method requires micronization of active ingredient with the disintegrant, spray coating of neutral granules with micronized drug-disintegrant mix followed by spray coating with polymer solution, which is a cumbersome process.

## SUMMARY OF THE INVENTION

By expenditure of considerable intellectual effort and careful experimentation the inventors have discovered that nimesulide can be formulated into a controlled release once-a-day oral dosage form.

Such dosage forms provide extended release of nimesulide in-vivo when given once daily with reproducible bioavailability. Further the release of such dosage forms is not effected by pH changes in the gastrointestinal system.

The composition in accordance with present invention comprises a controlled release pharmaceutical composition of Nimesulide which comprises nimesulide as an active drug from 5% to 95% w/w of the composition, one or more release sustaining materials from 2% to 95% w/w of the composition and pharmaceutical excipients from 0% to 90% w/w of the composition, in the form of a bilayer tablet with a first layer which gives fast release of the drug, and a second layer which gives extended release of the drug.

Preferably the composition in accordance with the present invention comprises nimesulide as an active drug from 20% to 70% w/w of the composition, one or more sustaining materials from 5% to 65% w/w of the composition and pharmaceutical excipients from 10% to 70% w/w of the composition.

More preferably the composition in accordance with the present invention comprises nimesulide as an active drug from 40% to 60% w/w of the composition, one or more sustaining materials from 8% to 20% w/w of the composition and pharmaceutical excipients from 30% to 60% w/w of the composition.

## DETAILED DESCRIPTION OF INVENTION

In accordance with the present invention there is disclosed a controlled release composition of Nimesulide.

The composition in accordance with present invention comprises a controlled release pharmaceutical composition of Nimesulide which comprises nimesulide as an active drug from 5% to 95% w/w of the composition, one or more sustaining materials from 2% to 95% w/w of the composition and pharmaceutical excipients from 0% to 90% w/w of the composition. In another aspect, such compositions contain nimesuilde in micronized form having average particle size below 5 microns.

Preferably the composition in accordance with the present invention comprises nimesulide as an active drug from 20% to 70% w/w of the composition, one or more sustaining materials from 5% to 65% w/w of the composition and pharmaceutical excipients from 10% to 70% w/w of the composition.

More preferably the composition in accordance with the present invention comprises nimesulide as an active drug from 40% to 60% w/w of the composition, one or more sustaining materials from 8% to 20% w/w of the composition and pharmaceutical excipients from 30% to 60% w/w of the composition.

In a preferred embodiment of the invention the composition consists of bilayer tablets wherein the active agent may be present in one or both layers. The bilayer tablets may be coated or uncoated. The coating may be semi-permeable type membrane. Further, the semi-permeable coat may have an orifice drilled through it on the drug layer side to provide passage for constant release of drug.

In another aspect of the invention the coating may be of microporous type through which the drug release takes place at constant rate.

The first fast release layer comprises materials like disintegrants, fitters, rapidly soluble/dispersible excipients and wetting agents. The second extended release layer comprises sustaining polymers, binders, wetting agents and fillers.

The sustaining polymers preferably are hydrophilic in nature and present in a blend of fast and slow hydrating polymers.

The sustaining materials ane selected from the group comprising cellulose and cellulose derivatives, waxes, carbomers, polyalkylene polyols, polycarbophils, methacrylate acid derivatives, gelatins, gums, polyethylene oxides.

The sustaining materials comprise materials which are non-toxic and pharmaceutically acceptable. These may be natural, semi-synthetic, synthetic or man-modified. Suitable materials include cellulose and cellulose derivatives like microcrystalline cellulose, methyl cellulose, ethyl cellulose, hydroxypropylmethyl cellulose, hydroxypropyl cellulose, cellulose acetate phthalate, cellulose acetate, cellulose acetate butyrate, cellulose acetate propionate, cellulose acetate trimellitate, cellulose carboxymethyl ethers and their salts, hydroxypropylmethyl cellulose phthalate, hydroxypropylmethyl cellulose acetate succinate.

Polyethylene; Polyquaternium-1; Polyvinyl acetate (homopolymer); Polyvinyl acetate phthalate; Propylene glycol alginate; Polyvinylmethacrylate/maleic anhydride (PVM/MA) copolymer; Polyvinylpyrollidone (PVP)/ dimethiconylacrylate/polycarbamyl/polyglycolester;PVP/dimethylaminoethyl methacrylate copolymer; PVP/dimethylaminoethyl methacrylate/polycarbamyl polyglycol ester; PVP/polycarbamyl polyglycol ester; Polyvinylpyrollidone/vinyl acetate (PVPNA) copolymer

Lanolin and lanolin derivatives, glyceryl monostearate, stearic acid, paraffins, beeswax, carnauba wax, Tribehenin.

Polyalkylene polyols like polyethylene glycols.

Gelatin and gelatin derivatives.

Alginates, Carbomers, Polycarbophils.

Methacrylic acid copolymers.

Carrageenans, pectins, chitosans, cyclodextrins, lecithins.

Natural and synthetic gums containing galactomannans like xanthan gum, tragacanth, acacia, agar, guar gum, etc.

Pharmaceutical excipients as used in the composition are selected from the group of excipients generally used by persons skilled in the art e.g. fillers, bulking agent, colourants, stabilizers, preservatives, lubricants, glidants, chelating agents and the like.

Preferably the composition also comprises release modifiers. Such release modifiers are selected from the groups comprising wetting agents, solubilizers, surfactants, plasticizers, solvents, pore formers, pH modifiers and tonicity adjusting agents.

Suitable example of such ingredients include:

Reaction products of natural and hydrogenated vegetable oils and ethylene glycol e.g. polyoxyethylene glycolated natural or hydrogenated castor oil such as those available under the trade name Cremophor.

Other suitable products include polyoxyethylene sorbitan fatty acid esters e.g. of the type available under the trade name TWEEN.
Polyoxyethylene fatty acid esters e.g. MYRJ and CETIOL HE.
Polyoxyethylene polyoxypropylene copolymers e.g. PLURONIC and
Polyoxyethylene polyoxypropylene block copolymers e.g. POLOXAMER.
Dioctylsodiumsulfosuccinate, sodium lauryl sulphate.
Propylene glycol mono- and di- fatty acid esters e.g. MIGLYOL 840.
Bile salts e.g alkali metals salts e.g. sodium taurocholate.
Polyethylene glycols, propylene glycol, triacetin, diacetin, diethyl phthalate, dibutyl phthalate, castor oil, triethyl citrate dibutyl sebacate.
Sodium chloride, potassium chloride, lactose, mannitol, sucrose, sorbitol.
Sodium hydroxide, potassium hydroxide, sodium bicarbonate, sodium citrate, citric acid, hydrochloric acid, lactic acid, tartaric acid, malic acid.

The calculation of dose of nimesulide for once-a-day controlled release dosage form was done on the basis of its pharmacokinetic parameters using the following equation :

$$\text{Dose} = C_P \times V_d \times K_{el} \times T$$

$C_P$ = Effective plasma concentration, 3.0 mg/L
$V_d$ = Apparent Volume of distribution, 15.6 L
$K_{el}$ = Elimination Rate constant, 0.166 $h^{-1}$
T = Desired Duration of action, 24 hrs

Based on the above equation the dose was calculated to be 207.0 mg. The compositions of the present invention have another added advantage that once - a - day dosage form of Nimesulide may be combined with another suitable long - acting drug to have synergistic activity. The other drug may be present in non-controlled release form. Such drugs may be selected from following categories :

(i) Antihistaminics e.g. Cetirizine Dihydrochloride.
(ii) Antispasmodics e.g. Pitofenone Hydrochloride, Hyoscine Hydrobromide.
(iii) Antiasthmatics e.g. Ketotifen, Salbutamol.

The following examples include both reference examples showing controlled release compositions of nimesulide in general (Examples 1-9) and an illustrative embodiment of the invention (Example 10) which is merely exemplary.

**Example 1** Controlled release matrix tablet type

| | | |
|---|---|---|
| (i) Nimesulide (micronized) | - | 200 mg |
| (ii) Lactose | - | 73 mg |
| (iii) Hydroxypropylmethyl Cellulose | - | 70 mg |
| (iv) Magnesium Stearate | - | 3.5 mg |
| (v) Purified Talc | - | 3.5 mg |

Blend (i), (ii), (iii), (iv) and (v) after sifting through mesh no. 30 (BSS). Compress into tablets.

The results of Dissolution Release Profile of Nimesulide CR Tablets based on example 1 are given below :

**Table 1**

| Time | Mean | SD |
|---|---|---|
| 30 mins. | 4.2 | ± 1.36 |
| 1 hr | 7.9 | ± 1.02 |
| 2 hrs | 16.4 | ± 1.74 |
| 3 hrs | 25.8 | ± 1.28 |

(continued)

| Time | Mean | SD |
|---|---|---|
| 4 hrs | 34.2 | ± 1.71 |
| 6 hrs | 50.8 | ± 2.44 |
| 8 hrs | 65.9 | ± 1.86 |
| 10 hrs | 74.9 | ± 0.97 |
| 12 hrs | 85.8 | ± 2.34 |
| 14 hrs | 93.5 | ± 2.49 |
| 16 hrs | 96.7 | ± 2.16 |
| 18 hrs | 97.1 | ± 1.08 |
| 19 hrs | 98.8 | ± 1.32 |

The dissolution profile as given in table 1 of the nimesulide sustained release tablet should not be construed to limit the scope of the invention. Variations to the dissolution profile can be possible depending upon the dosage requirements without departing from the spirit of the invention.

**Example 2** Extended release membrane diffusion controlled tablet type

| | | |
|---|---|---|
| (i) Nimesulide (micronized) | - | 200 mg |
| (ii) Microcrystalline Cellulose | - | 60 mg |
| (iii) Lactose | - | 60 mg |
| (iv) Maize Starch | - | 10 mg |
| (v) Purified Talc | - | 3.5 mg |
| (vi) Ethyl Cellulose (As Aqueous Dispersion) | - | 10 mg |
| (vii) Polyethylene Glycol | - | 3.5 mg |

Blend (i), (ii) and (iii) and granulate with starch paste and dry the granules. Sift through mesh no. 22 (BSS). Lubricate with Talc. Compress into tablets. Coat the tablets with Ethyl Cellulose using Polyethylene Glycol as a channel former.

**Example 3** Sustained release bead type

| | | |
|---|---|---|
| (i) Non - Pareil Beads | - | 347 mg |
| (ii) Nimesulide | - | 200 mg |
| (iii) Mannitol | - | 30 mg |
| (iv) Lactose | - | 30 mg |
| (v) Polyvinyl Pyrrolidone | - | -20 mg |
| (vi) Purified Talc | - | 15 mg |
| (vii) Ethyl Cellulose | - | 7 mg |
| (viii) Diethyl Phthalate | - | 1.4 mg |

Coat the non-pareil beads with blend of (ii), (iii) and (iv) using (v) as a binder in a conventional or fluidized bed coater. Talc may be dusted onto the beads. Final coating is given with Ethyl Cellulose using (viii) as plasticizer.

**Example 4** Osmotically controlled constant release type device

| Upper Layer | | |
|---|---|---|
| (i) Nimesulide (micronized) | - | 200 mg |
| (ii) Sodium Hydroxide | - | 15 mg |
| (iii) Lactose | - | 34 mg |
| (iv) Sodium Chloride | - | 30 mg |
| (v) Polyvinyl Pyrrolidone | - | 6 mg |
| (vi) Polyethylene Oxide | - | 1.5 mg |
| **Lower Layer** | | |
| (vii) Polyethylene Oxide | - | 22 mg |
| (viii) Hydroxypropylmethyl Cellulose | - | 1.8 mg |
| (ix) Sodium Chloride | - | 20 mg |
| (x) Dichloromethane | - | q.s (Lost in processing) |
| **Semi-permeable Coat** | | |
| (xi) Cellulose Acetate | - | 30 mg |
| (xii)Triacetin | - | 1 mg |
| (xiii) Acetone | - | q.s (Lost in procesing) |
| (xiv) Water | - | -q.s (Lost in processing) |

Blend finely powdered (i), (ii), (iii), (iv) and (vi). Granulate with aqueous solution of (v). Granulate the blend of (vii) and (ix) with dispersion of (viii) in (x). Compress the two granulates into bilayer tablets and coat with the dispersion of (xii) and (xiii) in aqueous acetone. Finally, drill a hole in the drug layer (Upper layer) through which the drug is released in a controlled fashion due to osmotic pressure.

The results of Dissolution Release Profile of Nimesulide CR Tablets based on example 4 are given below :

Table 2

| Time | Mean | SD |
|---|---|---|
| 2 hours | 5.16 | ± 0.53 |
| 4 hours | 16.75 | ± 1.68 |
| 6 hours | 34.90 | ± 2.26 |
| 8 hours | 45.75 | ± 2.26 |
| 10 hours | 56.00 | ± 4.36 |
| 12 hours | 67.85 | ± 4.40 |
| 14 hours | 79.16 | ± 5.03 |
| 14 hours | 90.25 | ± 3.68 |
| 18 hours | 101.16 | ± 3.53 |

**Example 5** Coated capsule type

| | | |
|---|---|---|
| (i) Nimesulide (micronized) | - | 200 mg |
| (ii) Microcrystalline Cellulose | - | 88.4 mg |
| (iii) Lactose | - | 70 mg |
| (iv) Polyvinyl Pyrrolidone | - | 7 mg |
| (v) Magnesium Stearate | - | 3.9 mg |
| (vi) Ethyl Cellulose | - | 20 mg |
| (vii) Polyethylene Glycol | - | 0.7 mg |
| (viii) Alcohol : Dichloromethane (1 : 2) | - | q.s (Lost in processing) |
| (ix) Empty Gelatin Capsule (Size '1') | | |

Blend (i), (ii), (iii), (iv) and (v) and fill into empty gelatin capsule size '1'. Coat the capsules with dispersion of (vi) and (vii) in (viii).

**Example 6** pH dependent delayed release type

| | | |
|---|---|---|
| (i) Nimesulide (micronized) | - | 100 mg |
| (ii) Microcrystalline Cellulose | - | 150 mg |
| (iii) Lactose | - | 76 mg |
| (iv) Polyoxyl 40 Hydrogenated Castor Oil | - | 7 mg |
| (v) Polyvinyl Pyrrolidone | - | 10 mg |
| (vi) Magnesium Stearate | - | 3.5 mg |
| (vii) Purified Talc | - | 3.5 mg |
| (viii) Cellulose Acetate Phthalate | - | 28 mg |
| (ix) Diethyl Phthalate | - | 2 mg |
| (x) Water | - | q.s (Lost in processing) |
| (xi) Alcohol : Dichloromethane (1:2) | - | q.s (Lost in processing) |

Granulate the blend of (i), (ii) and (iii) with solution of (iv) and (v) in water. Blend the granules with (vi) and (vii). Compress into tablets. Coat with the dispersion of (viii) and (ix) in (xi).

**Example 7** Timed release bead type

| | | | |
|---|---|---|---|
| (i) Nimesulide (micronized) | 100 mg | 100 mg | 100 mg |
| (ii) Microcrystalline Cellulose | 200 mg | 200 mg | 200 mg |
| (iii) Lactose | 50 mg | 42 mg | 35mg |
| (iv) Polyvinyl Pyrrolidone | 10 mg | 10 mg | 10 mg |
| (v) Water | q.s | q.s | q.s |
| (vi) Ammonio Methacrylate Copolymer Type B (Eudragit RS) | 10 mg | 18 mg | 25 mg |
| (vii) Diacetin | 0.5 mg | 0.5 mg | 0.5 mg |
| (viii) Water: Acetone (1:9) | q.s | q.s | q.s |

Procedure :

In this composition 3 types of beads are prepared which are coated with different amounts of (vi) to give a timed profile of the drug. Beads are prepared by blending and spheronizing (I), (ii) and (iii) jusing aqueous solution of (iv). The dried beads are coated with dispersion of (vi) and (vii) in (viii). The 3 different beads are blended together in a fixed ratio to obtain the required release profile.

**Example 8 Osmotically controlled constant release system**

| Active Layer | | |
|---|---|---|
| (i) Nimesulide (micronized) | - | 200.0 mg |
| (ii) Polyethylene oxide | - | 116.5 mg |
| (iii) Hydroxypropylmethyl cellulose | - | 10.0 mg |
| (iv) Sodium chloride | - | 10.0 mg |
| (v) Magnesium stearate | - | 2.5 mg |
| **Push layer** | | |
| (vi) Polyethylene oxide | - | 140.0 mg |
| (vii) Sodium chloride | - | 50.0 mg |

(continued)

| Push layer | | |
|---|---|---|
| (viii) Hydroxypropylmethy cellulose | - | 9.5 mg |
| (ix) Magnesium stearate | - | 0.5 mg |
| (x) Iron oxide red | - | 1.0 mg |
| Functional coating | | |
| (xi) Cellulose acetate | - | 45.0 mg |
| (xii) Polyethylene glycol | - | 5.0 mg |
| (xiii) Acetone | - | Lost in processing |
| Non-functional coating | | |
| (xiv) Titanium dioxide | - | 2.0 mg |
| (xv) Hydroxypropylmethyl cellulose | - | 6.0 mg |
| (xvi) Purified Talc | - | 2.0 mg |
| (xvii) Polyethylene glycol - 400 | - | 2.0 mg |
| (xviii) Isopropyl Alcohol | - | Lost in processing |
| (xix) Dichloromethane | - | Lost in processing |

Procedure : Blend (I), (ii), (iii), (iv) and (v) in a double cone blender. Separately blend (vi), (vii), (viii) (ix) and (x). Compress into bilayer tablet using a suitable compression machine. Coat the tablets with the dispersion of (xi) and (xii) in (xiii). The tablets are further coated with the dispersion of (xiv), (xv), (xvi), (xvii) in mixture of (xviii) and (xix).

**Example 9 : Bilayer tablets having one fast release layer and one extended release layer**

| Fast Release layer | | |
|---|---|---|
| (i) Nimesulide (micronized) | - | 100.0 mg |
| (ii) Lactose | - | 151.5 mg |
| (iii) Starch | - | 37.6 mg |
| (iv) Colloidal silicon Dioxide | - | 11.0 mg |
| (v) Povidone K-30 | - | 8.5 mg |
| (vi) Docusate Sodium | - | 6.8 mg |
| (vii) Polysorbate 80 | - | 1.0 g |
| (viii) Magnesium Stearate | - | 1.6 mg |
| (ix) Croscarmellose Sodium | - | 22.0 mg |
| (x) Water - | - | Lost in processing |
| Extended Release Layer | | |
| (xi) Nimesulide (micronized) | - | 100.0 mg |
| (xii) Lactose | - | 200.0 mg |
| (xiii) Hydroxypropylmethyl cellulose K100LV | - | 23.0 mg |
| (xiv) Hydroxypropylmethyl cellulose K4MCR | - | 100.0 mg |
| (xv) Povidone K-30 | - | 9.0 mg |
| (xvi) Docusate Sodium | - | 4.5 mg |
| (xvii) Magnesium Stearate | - | 4.5 mg |
| (xviii) Colloidal Silicon Dioxide | - | 4.5 mg |
| (xix) Sodium Lauryl Sulphate | - | 4.5 mg |
| (xx) Isopropyl Alcohol | - | Lost in processing |

Procedure:

Blend 1. : Blend (i), (ii), (iii) and (iv) and granulate with solution of (v) and (vi) in (x). Dry the granules and blend with (vii), (viii) and (ix).

Blend 2 : Blend (xi), (xii), (xiii) and (xiv) and granulate with solution of ((xv) and (xvi) in (xx). Dry the granules and mix with (xvii), (xviii) and (xix).

Compress into bilayer tablets using a suitable compression machine.

**Example 10 : Bilayer tablets having one fast release layer containing drug in complexed form and one extended release layer**

| A. Fast Release layer | | |
|---|---|---|
| (i) Nimesulide (micronized) | - | 100.0 mg |
| (ii) β-cyclodextrin | - | 400.0 mg |
| (iii) Starch | - | 70.0 mg |
| (iv) Povidone K-30 | - | 7.5 mg |
| (v) Croscarmellose Sodium | - | 20.0 mg |
| (vi) Magnesium Stearate | - | 2.5 mg |
| B. Extended Release Layer | | |
| (vii) Nimesulide (micronized) | - | 100.0 mg |
| (viii) Lactose | - | 200.0 mg |
| (ix) Hydroxypropylmethyl cellulose K100LV | - | 23.0 mg |
| (x) Hydroxypropylmethyl cellulose K4MCR | - | 100.0 mg |
| (xi) Povidone K-30 | - | 9.0 mg |
| (xii) Magnesium Stearate | - | 4.5 mg |
| (xiii) Colloidal Silicon Dioxide | - | 4.5 mg |
| (xiv) Docusate Sodium | - | 4.5 mg |

Procedure :

Layer - I

1. Mix (i) and (ii), co-mill under specific conditions favouring complexation using ball mill to prepare a complex.
2. Mix complex of step 1 with (iii) and granulate with a solution of (iv) in water.
3. Dry the granules at 40° - 50°C.
4. Size the granules & mix with (v) and (vi)

Layer - II

1. Mix (vii), (viii), (ix) and (x). Granulate with a solution of (xi) and (xiv).
2. Dry the granules at 40° - 50°C.
3. Size the granules & mix with (xii) and (xiii).
4. Compress the two layers into bilayered tablets using suitable compression machine.

## Claims

**1.** A compressed controlled release pharmaceutical composition of nimesulide which comprises nimesulide as an active drug from 5% to 95% w/w of the composition, one or more release sustaining materials from 2% to 95% w/w of the composition and pharmaceutical excipients from 0% to 90% w/w of the composition, in the form of bilayer tablet with a first layer which gives fast release of the drug and a second flayer which gives extended release of the drug.

**2.** A controlled release pharmaceutical composition of nimesulide as claimed in claim 1 which comprises nimesulide as an active drug from 20% to 70% w/w of the composition, one or more sustaining materials from 5% to 65% w/w of the composition and pharmaceutical excipients from 10% to 70% w/w of the composition.

3. A controlled release pharmaceutical composition of nimesulide as claimed in claim 1 which comprises nimesulide as an active drug from 40% to 60% w/w of the composition, one or more sustaining materials from 8% to 20% w/w of the composition and pharmaceutical excipients from 30% to 60% w/w of the composition.

4. A controlled release pharmaceutical composition of nimesulide as claimed in claims 1 to 3, wherein the sustaining materials are selected form the group comprising cellulose and cellulose derivatives, waxes, carbomers, polyalkylene polyols, polycarbophils, methacrylic acid derivatives, gelatins, gums and polyethylene oxides.

5. A composition as claimed in claims 1 to 4 which further comprises release modifiers selected form the group comprising wetting agents, solubilizers, surfactants, plasticizers, pore formers, pH modifiers and tonicity adjusting agents.

6. A process for the manufacture of a controlled release pharmaceutical composition of nimesulide as claimed in claim 1 which comprises mixing together and compressing nimesulide as an active drug from 5% to 95% w/w of the composition, one or more release sustaining materials from 2% to 95% w/w of the composition and pharmaceutical excipients from 0% to 90% w/w of the composition, into a bilayer tablet with a first layer which gives fast release of the drug and a second flayer which gives extended release of the drug.

## Patentansprüche

1. Komprimierte pharmazeutische Zusammensetzung von Nimesulid mit kontrollierter Freisetzung, enthaltend 5 Gew.-% bis 95 Gew.-%, bezogen auf die Zusammensetzung, Nimesulid als aktiven Wirkstoff, 2 Gew.-% bis 95 Gew.-%, bezogen auf die Zusammensetzung, eines oder mehrerer freisetzungsverzögernder Materialien und 0 Gew.-% bis 90 Gew.-%, bezogen auf die Zusammensetzung, pharmazeutische Exzipienten, in Form einer Zwei-Schicht-Tablette mit einer ersten Schicht, die den Wirkstoff schnell freisetzt, und einer zweiten Schicht, die den Wirkstoff retardiert freisetzt.

2. Pharmazeutische Zusammensetzung von Nimesulid mit kontrollierter Freisetzung nach Anspruch 1, enthaltend 20 Gew.-% bis 70 Gew.-%, bezogen auf die Zusammensetzung, Nimesulid als aktiven Wirkstoff, 5 Gew.-% bis 65 Gew.-%, bezogen auf die Zusammensetzung, eines oder mehrerer freisetzungsverzögernder Materialien und 10 Gew.-% bis 70 Gew.-%, bezogen auf die Zusammensetzung, pharmazeutische Exzipienten.

3. Pharmazeutische Zusammensetzung von Nimesulid mit kontrollierter Freisetzung nach Anspruch 1, enthaltend 40 Gew.-% bis 60 Gew.-%, bezogen auf die Zusammensetzung, Nimesulid als aktiven Wirkstoff, 8 Gew.-% bis 20 Gew.-%, bezogen auf die Zusammensetzung, eines oder mehrerer freisetzungsverzögernder Materialien und 30 Gew.-% bis 60 Gew.-%, bezogen auf die Zusammensetzung, pharmazeutische Exzipienten.

4. Pharmazeutische Zusammensetzung von Nimesulid mit kontrollierter Freisetzung nach einem der Ansprüche 1 bis 3, wobei die freisetzungsverzögernden Materialien ausgewählt sind unter Zellulose und Zellulosederivaten, Wachsen, Carbomeren, Polyalkylenpolyolen, Polycarbophilen, Methacrylsäurederivaten, Gelatine, Gummis und Polyethylenoxiden.

5. Zusammensetzung nach den Ansprüchen 1 bis 4, außerdem umfassend Freisetzungsmodifikatoren, ausgewählt unter Netzmitteln, Lösungsvermittlern, oberflächenaktiven Substanzen, Weichmachern, Porenbildnern, pH-Modifikatoren und tonizitätsverändernden Substanzen.

6. Verfahren zur Herstellung einer pharmazeutischen Zusammensetzung von Nimesulid mit kontrollierter Freisetzung nach Anspruch 1, umfassend das Mischen und Komprimieren von 5 Gew.-% bis 95 Gew.-%, bezogen auf die Zusammensetzung, Nimesulid als aktiven Wirkstoff, 2 Gew.-% bis 95 Gew.-%, bezogen auf die Zusammensetzung, eines oder mehrerer freisetzungsverzögernder Materialien und 0 Gew.-% bis 90 Gew.-%, bezogen auf die Zusammensetzung, pharmazeutischer Exzipienten, zu einer Zwei-Schicht-Tablette mit einer ersten Schicht, die den Wirkstoff schnell freisetzt, und einer zweiten Schicht, die den Wirkstoff retardiert freisetzt.

## Revendications

1. Composition pharmaceutique comprimée de nimésulide à libération contrôlée, comprenant du nimésulide en tant

que médicament actif de 5 % à 95 % p/p de la composition, une ou plusieurs matières de libération soutenue de 2 % à 95 % p/p de la composition et des excipients pharmaceutiques de 0 % à 90 % p/p de la composition, sous la forme d'une pastille bicouche, une première couche offrant une libération rapide du médicament et une deuxième couche offrant une libération prolongée du médicament.

**2.** Composition pharmaceutique de nimésulide à libération contrôlée selon la revendication 1, comprenant du nimésulide en tant que médicament actif de 20 % à 70 % p/p de la composition, une ou plusieurs matières de libération soutenue de 5 % à 65 % p/p de la composition et des excipients pharmaceutiques de 10 % à 70 % p/p de la composition.

**3.** Composition pharmaceutique de nimésulide à libération contrôlée selon la revendication 1, comprenant du nimésulide en tant que médicament actif de 40 % à 60 % p/p de la composition, une ou plusieurs matières de libération soutenue de 8 % à 20 % p/p de la composition et des excipients pharmaceutiques de 30 % à 60 % p/p de la composition

**4.** Composition pharmaceutique de nimésulide à libération contrôlée selon l'une quelconque des revendications 1 à 3, dans laquelle les matières de libération soutenue sont choisies dans le groupe comprenant la cellulose et les dérivés de la cellulose, les cires, les carbomères, les polyalkylène polyols, les polycarbophiles, les dérivés de l'acide méthacrylique, les gélatines, les gommes et les oxydes de polyéthylène.

**5.** Composition selon l'une quelconque des revendications 1 à 4, comprenant en outre des modificateurs de libération choisis dans le groupe comprenant les agents mouillants, les agents de solubilisation, les tensioactifs, les plastifiants, les agents de formation de pores, les modificateurs de pH et les agents d'ajustement de la tonicité.

**6.** Procédé de fabrication d'une composition pharmaceutique de nimésulide à libération contrôlée selon la revendication 1, comprenant le mélange ensemble et la compression du nimésulide en tant que médicament actif de 5 % à 95 % p/p de la composition, d'une ou de plusieurs matières de libération soutenue de 2 % à 95 % p/p de la composition et des excipients pharmaceutiques de 0 % à 90 % p/p de la composition, en une pastille bicouche, une première couche offrant une libération rapide du médicament et une deuxième couche offrant une libération prolongée du médicament.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5688829 A
- US 5716609 A
- CA 2253061
- WO 9912524 A
- US 5788987 A, Busetti
- ES 2129010


### Non-patent literature cited in the description


- **Magni E.** Nimesulide an overview. *Drug Invest,* 1991, vol. 3 (2), 1-3
- **Magni E.** The effect of nimesulide on prostanoid formation. *Drugs,* 1993, vol. 46 (1), 10-4
- *Drugs,* 1994, vol. 48 (3), 431-454